(19) Europäisches Patentamt
European Patent Office
Office européen des brevets

(11) **EP 1 140 285 B1**

(12) **EUROPÄISCHE PATENTSCHRIFT**

(45) Veröffentlichungstag und Bekanntmachung des Hinweises auf die Patenterteilung:
**17.11.2004   Patentblatt 2004/47**

(51) Int Cl.$^7$: **A61N 1/365**

(21) Anmeldenummer: **99962249.1**

(22) Anmeldetag: **30.11.1999**

(86) Internationale Anmeldenummer:
**PCT/EP1999/009756**

(87) Internationale Veröffentlichungsnummer:
**WO 2000/040296 (13.07.2000 Gazette 2000/28)**

(54) **HERZSCHRITTMACHER**

CARDIAC PACEMAKER

STIMULATEUR CARDIAQUE

(84) Benannte Vertragsstaaten:
**CH DE FR IT LI**

(30) Priorität: **05.01.1999   DE 19900690**

(43) Veröffentlichungstag der Anmeldung:
**10.10.2001   Patentblatt 2001/41**

(73) Patentinhaber: **St. Jude Medical AB**
**175 84 Järfälla (SE)**

(72) Erfinder:
 • **HEINZE, Roland**
   **D-14059 Berlin (DE)**
 • **STANGL, Karl**
   **D-10709 Berlin (DE)**

(56) Entgegenhaltungen:
**US-A- 4 399 820          US-A- 4 870 968**
**US-A- 5 500 006**

## Beschreibung

Die Erfindung betrifft einen Herzschrittmacher nach dem Oberbegriff des Hauptanspruchs.

Ein allgemein bekannter Herzschrittmacher ist der sogenannte QT- oder Stimulus-T-Schrittmacher wie er beispielsweise in der US 422 8 803 beschrieben ist. Ein solcher Herzschrittmacher weist Mittel auf, mit denen die mittlere Stimulationsfrequenz an Änderungen der physischen und psychischen Belastung angepaßt werden kann.

Hierzu ist ein Schaltkreis vorgesehen, der das intrakardial abgeleitete EKG-Signal auswertet, wobei er den Beginn oder das Maximum der T-Welle detektiert. Da sich der zeitliche Abstand zwischen Stimulation und Einsetzen der T-Welle das sogenannte Stim-T-Intervall bei steigender Belastung verkürzt, liefert der Schaltkreis einen physiologischen Meßparameter, mit dem die Stimulationsfrequenz sich ändernden Belastungen angepaßt werden kann.

Der prinzipielle Nachteil einer derartigen Frequenzregelung ist dadurch gegeben, daß sich das Stim-T-Intervall nicht nur bei Anstieg der Belastung sondern in weitaus stärkerem Maße durch den Anstieg der Stimulationsfrequenz selbst verkürzt. Eine derartige Frequenzregelung erfordert dementsprechend spezielle Maßnahmen, um eine positive Rückkoppelung zu vermeiden.

Ein weiterer Nachteil dieser Frequenzregelung ist die Tatsache, daß die gemessenen Stim-T-Intervalle humoral bestimmt sind d.h. aufgrund der über die Nebennierenrinde ausgeschütteten, und über den Blutkreislauf transportierten Hormone reagieren.

Grundsätzlich ist bei der Regelung der Stimulationsfrequenz in Herzschrittmachern ein wesentliches Ziel, die Stimulationsfrequenz nicht nur an steigende körperliche Belastungen anzupassen, sondern dabei auch die individuelle myokardiale Leistungsfähigkeit des Patienten zu berücksichtigen. Das bedeutet, daß die Stimulationsfrequenz bei steigender Belastung nur solange erhöht wird, wie dadurch eine Steigerung des Herzzeitvolumens HZV erreicht wird. Damit soll verhindert werden, daß der Herzmuskel durch eine zu hohe Stimulationsfrequenz ("Overpacing") überlastet und geschädigt wird.

Es wurde versucht, diese Kontrolle durch Messung des Schlagvolumens SV oder eines HZV-abhängigen Meßparameters, wie z.B. der zentralvenösen Sauerstoffsättigung (sO2) zu erreichen.

Aus der WO 89/06990 ist ein Verfahren zur hämodynamischen Optimierung der Stimulationsfrequenz bekannt, das die Messung der vom Herzzeitvolumen HZV-abhängigen zentralvenösen Sauerstoffsättigung sO2 in Kombination mit einer Modulation der Stimulationsfrequenz $\Delta$HR über Phasen von 2 bis 4 Minuten benutzt. Eine Optimierung des Herzzeitvolumens wird dadurch angestrebt, daß der frequenzabhängige Gradient der Sauerstoffsättigung $\Delta$sO2/$\Delta$HR innerhalb eines vorgegebenen Bereichs gehalten wird, der analog zum Gradienten des Herzzeitvolumens HZV/$\Delta$HR ein physiologisch optimaler Bereich ist.

Dieses Verfahren ist von der Stabilität und der Genauigkeit des sO2-Sensorkatheters abhängig, die sich in der Praxis als nicht ausreichend erwiesen haben und es weist den Nachteil auf, daß es wegen der notwendig langen Änderungsperioden nicht in der erforderlichen Zeit von einigen Minuten gelingt zu differenzieren, ob die gemessene sO2-Änderung durch die Frequenzänderung oder durch andere Einflußgrößen bewirkt wurde.

Die EP 0 551 355 beschreibt ein Verfahren zur Modulation einzelner Stimulationsintervalle, bei dem die Impedanzmessung zur Erfassung des Schlagvolumens genutzt wird, um den Einsatz eines Sensorkatheters zur Bestimmung des Herzzeitvolumens zu umgehen. Durch die gezielte Modulation einzelner Stimulationsintervalle $\Delta$SI und die phasenspezifische Demodulation der Impedanzänderung $\Delta$Z wurde angestrebt, den Einfluß nicht funktionsspezifischer und somit störender Parameteränderungen zu unterdrücken und zusätzlich das Signal mit Hilfe maximaler Modulation kalibriert.

Dieses Verfahren weist den Nachteil auf, daß das Prinzip der Modulation einzelner Stimulationsintervalle hier nur als Filter- und Kalibrierverfahren, d.h. als Zwischenschritt zur Bestimmung des Schlagvolumens und damit des Herzzeitvolumens HZV genutzt wird. Die Optimierung der Frequenzregelung wird dann gleichfalls durch die Optimierung des Gradienten $\Delta$HZV/$\Delta$HR anhand einer optimalen hämodynamischen Kennlinie angestrebt. Die Bestimmung des Schlagvolumens hat sich trotz Verbesserung des Störabstandes durch die Einzelpulsmodulation in der Praxis als noch zu ungenau erwiesen, um eine zuverlässige hämodynamische Optimierung durchführen zu können. Das heißt, daß eine Optimierung der Stimulation durch Kontrollierung des Herzzeitvolumens sich in der Praxis als problematisch darstellt, da entweder die spezifischen Sensorkatheter zur Messung des Schlagvolumens oder HZVabhängiger Meßparameter noch keine ausreichende Langzeitstabilität aufweisen oder Messungen des Schlagvolumens mit Standardkathetern über die Impedanz nicht zuverlässig genug sind. Außerdem wird die Auswertung sehr komplex da die das Meßergebnis verfälschenden und miterfaßten mechanischen Übertragungsfunktionen berücksichtigt werden müssen.

Der Erfindung liegt die Aufgabe zugrunde, einen Herzschrittmacher zu schaffen, der eine schnelle und genaue Regelung der Stimulationsfrequenz bzw. der Stimulationsintervalldauer ermöglicht und eine Überlastung durch eine zu hohe Stimulationsfrequenz verhindert.

Diese Aufgabe wird erfindungsgemäß durch die Merkmale des Hauptanspruchs gelöst.

Der erfindungsgemäße Herzschrittmacher mit einer individuell optimierten Regelung der Stimulationsintervalldauer umgeht die Notwendigkeit, einen SV- bzw. HZV-abhängigen Meßparameter zu bestimmen und ermöglicht durch Auswertung der elektrischen Restitution bzw. des Gradienten der elektrischen Restitution mit Hilfe der standardmäßigen Erfassung des endokardialen EKG's eine Regelung der Stimulationsfrequenz bzw. der Stimulationsintervalldauer mittels eines Funktionsparameters des Herzens, der direkt den Belastungszustand des Patienten wiedergibt, wobei auch Veränderungen der Leistungsfähigkeit des Herzmuskels und akute Verschlechterungen der myokardialen Leistung bei der Frequenzanpassung berücksichtigt werden. Dabei wird die Modulation einzelner Stimulationsintervalle so durchgeführt, daß die mittlere eingestellte Intervalldauer sich nicht ändert.

Durch die in den Unteransprüchen angegebenen Maßnahmen sind vorteilhafte Weiterbildungen und Verbesserungen möglich.

Die Modulation der Stimulationsintervalle um einen positiven und negativen Wert wird sowohl kontinuierlich als auch im Abstand von mehreren Pulsen unter periodischer Wiederholung durchgeführt.

Es wurde gefunden, daß die elektrische Restitutionskurve, die durch Messung der Aktionspotentialdauer bestimmt wird, äquivalent zu der ist, die durch Messung des QT- oder des Stim-T-Intervalls des Elektrokardiogramms definiert wird.

Weiterhin hat sich gezeigt, daß die Analyse der last- und der frequenzabhängigen Modulation des Stim-T-Intervalls ausreichend zuverlässig ist, wenn die Modulation eines einzelnen Stimulationsintervalls die Ungleichungen ESI (Extrasystolisches Intervall) <600 ms bei $\Delta ESI/BCL \geq 10\%$ (BCL-basic cycle length).

Als Auswertegröße der elektrischen Restitution kann vorteilhafterweise eine dimensionslose Größe z.B. der Gradient (ERG) oder die relative Änderung der elektrischen Restitution verwendet werden, um eine lastabhängige Steuerung zu erzielen. Dies ist möglich, da dieser Gradient mit einem Anstieg der physischen Last fällt, während er mit steigender Stimulationsfrequenz steigt. Außerdem konnte gefunden werden, daß die Änderungsreaktion hauptsächlich auf einer Änderung der Zeitkonstanten der exponentiellen Restitutionsfunktion beruht und diese Zeitkonstante reagiert wesentlich schneller und stärker auf Änderungen der Last und der Frequenz als es das Stim-T-Intervall bei einer Regelung nach dem Stand der Technik tut.

Weiterhin ist die erfindungsgemäße Regelung gut anwendbar bei akuter Ischämie, da die elektrische Restitution die myokardialen Bedingungen widerspiegelt. Die Zeitkonstante der exponentiellen elektrischen Restitution und dabei der Gradient derselben steigt mit der Ischämie. Dies bewirkt erfindungsgemäß eine Reduktion der Stimulationsfrequenz.

Die Regelung nach der Erfindung durch eine Einzelpulsmodulation und Erfassung der elektrischen Restitution bewirkt eine schnelle und genaue Regelung der Stimulationsfrequenz, da die elektrische Restitution hauptsächlich durch einen neuronal gesteuerten, schnellen Reaktionsmechanismus gesteuert wird.

Ein Ausführungsbeispiel der Erfindung ist in der Zeichnung dargestellt und wird in der nachfolgenden Beschreibung näher erläutert.

Es zeigen

Fig. 1    den charakteristischen Verlauf einer elektrischen Restitutionskurve eines normalen, gesunden Myokards für die Ruhe- und für die Belastungsphase,

Fig. 2    Kennlinien für den elektrischen Restitutionsgradienten als Funktion der Stimulationsfrequenz in der Ruhe- und Belastungsphase,

Fig. 3    Kennlinien des Gradienten der elektrischen Restitution abhängig von der Stimulationsfrequenz beim Auftreten einer Ischämie und

Fig. 4    ein Blockschaltbild eines Ausführungsbeispiels des Herzschrittmachers nach der vorliegenden Erfindung.

Als elektrische Restitution wird die Abhängigkeit der Dauer des Aktionspotentials AP des Herzmuskels als Funktionsparameter von der Dauer der Diastole $t_d$ bezeichnet. Wird diese während eines einzelnen Herzzyklus' spontan geändert, beispielsweise durch eine Extrasystole, dann ändert sich das Aktionspotential bzw. dessen Dauer. Die Dauer des Aktionspotentials wird durch das Intervall zwischen dem Anfang der Stimulation und dem Zeitpunkt definiert, bei dem das Aktionspotential um 90% abgefallen ist, und sie nimmt ab, wenn das Zeitintervall zwischen zwei aufeinanderfolgenden Stimulationspulsen kleiner wird. Hier ist zwischen der APD-Änderung nach einem extrasystolischen Stimulationsintervall und der APD-Änderung nach einer Änderung der mittleren oder Basisherzfrequenz (HR=1/BCL) entsprechend dem Stand der Technik zu unterscheiden.

Dieses Änderungsverhalten nach einem extrasystolischen Stimulationsintervall läßt sich durch eine doppelt exponentielle Funktion beschreiben, die als elektrische Restitutionskurve ER bezeichnet wird.

Die elektrische Restitutionskurve (ERC) ist somit definiert als Funktion der Aktionspotentialdauer APD von der Zykluslänge eines vorhergehenden extrasystolischen Stimulationspulsintervalls ESI, d.h. eines einzelnen Stimulationspulsintervalls, das von der Basiszykluslänge (BCL), d.h. der mittleren Stimulationsintervalldauer um $\pm\Delta$ESI geändert wird und das der Diastole entspricht.

Die Funktion kann beschrieben werden als

$$\text{ER APD(ESI)} = \text{APD}_{P_1}*(1-A1*\exp(-t_d/T1)-A2*\exp(-t_d/T2))$$

Hierbei sind $\text{APD}_{P_1}$ der Plateauwert, A1 und T1 Amplitude und Zeitkonstante der schnellen Phase der Restitution und A2 und T2 die Amplitude und Zeitkonstante der langsamen Phase der Restitution.

Die Unterscheidung in der Näherungsgleichung zwischen einem langsamen und einem schnellen Anteil beim exponentiellen Anstieg der Restitutionskurve trägt der Tatsache Rechnung, daß Funktion des Herzmuskels bzw. der Herzmuskelzelle wie der Ionenaustausch an der Zellmembran bestimmt sind, d.h. sowohl durch schnelle, autonome Regelprozesse in der Zelle und dem umliegenden Gewebe als auch durch Regelprozesse, die das gesamte Herz-Kreislaufsystem betreffen und über das vegetative Nervensystem und die entsprechenden Drüsenfunktionen gesteuert werden.

Als Meßparameter zur Bestimmung der elektrischen Restitutionskurve wird, wie oben angedeutet, prinzipiell die Aktionspotentialdauer APD bestimmt, die durch spezielle Elektroden gemessen werden kann. Untersuchungen haben jedoch gezeigt, daß bei Messung des EKGs auch das sogenannte QT-Intervall, also die Intervalldauer zwischen der Q-Zacke und dem Ende der T-Welle des intrakardialen EKGs die gleiche Restitutioncharakteristik hat wie die APD. Bei Stimulation des Ventrikels durch einen Herzschrittmacher ist es zweckmäßiger, anstelle des QT-Intervalls als Meßintervall das Stim-T-Intervall STI, also den Abstand zwischen Stimulationspuls und T-Welle zu messen.

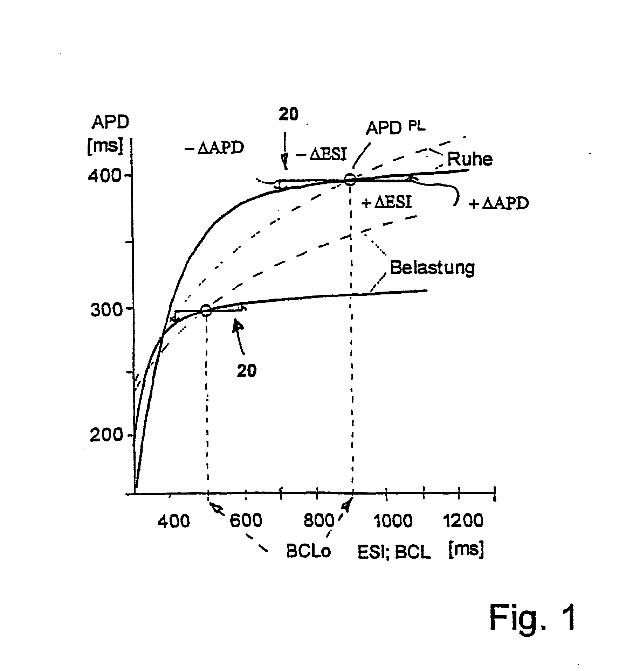

In FIG. 1 ist als elektrische Restitutionskurve (durchgezogene Linie) der Verlauf der Aktionspotentialdauer APD in Abhängigkeit von der Länge einzelner extrasystolischer Intervalle eines normalen, gesunden Myokards für die Ruhe- und für eine Belastungsphase dargestellt. Dabei wurden in beiden Phasen jeweils die optimal angepaßten Stimulationsfrequenz HRo bzw. der optimalen Basiszykluslänge BCLo = 1/HRo (d.h. die mittlere Stimulationsintervalldauer) in einzelnen extrasystolischen Stimulationsintervallen ESI verändert und dann die entsprechende Änderung der Aktionspotentialdauer APD gemessen. Die so entstandenen Restitutionskurven entsprechen den durch die obige Gleichung beschriebenen Exponentialfunktionen. Die optimale Basiszykluslänge BCLo für Ruhe (900 ms) und für eine Belastung (500 ms) sind durch die gestrichelten Pfeile bezeichnet, d.h. die jeweilige Basiszykluslängen bzw. mittlere Intervalldauer wurde um $\pm\Delta$ESI zur Bildung von extrasystolischen Intervallen geändert und jeweils als Reaktion die Aktionspotientaldauer bzw. das QT- oder das Stim-T-Intervall als Meßparameter gemessen. Dabei wird mittlere Stimulationsintervalldauer abwechselnd um positive und negative $\Delta$ESI-Werte so verlängert und verkürzt, daß die eingestellte mittlere Intervalldauer gleich bleibt. Vorzugsweise sind die $\pm\Delta$ESI bei einer Änderung gleich, d.h. die Intervalldauer wird um den gleichen Wert verkürzt und verlängert. Die Änderung kann sich im Abstand von mehreren Pulsen periodisch wiederholen, sie kann aber auch kontinuierlich, d.h. jeder Stimulationspuls wird abwechselnd verlängert oder verkürzt, durchgeführt werden.

Die gestrichelten Linien in FIG. 1 stellen die Kurven der QT- oder Stim-T-Intervalle eines EKGs bei kontinuierlicher Veränderung der Basiszykluslänge, bzw. bei kontinuierlicher Modulation, die beispielsweise bei einem QT-Schrittmacher nach dem Stand der Technik verwendet werden. Wie zu erkennen ist, unterscheiden sich diese Kennlinien deutlich von den elektrischen Restitutionskurven bei unterschiedlicher Belastung, wobei mit steigender Belastung neben einer Reduktion des Plateauwertes der jeweiligen Kurve mit entsprechender Verschiebung nach links auch ein steilerer Anstieg der Kurve gemessen wurde.

Die Restitutionskurve kann nun zur physiologischen Regelung der Stimulationsfrequenz HR genutzt werden, wobei, wie erwähnt, wesentlich ist, daß sowohl der Plateauwert $\text{APD}_{P_1}$ als auch die Zeitkonstanten T1 und T2 abhängig von der Pulsfrequenz HR und dem myokardialen Leistungspegel sind. Die Stimulationsfrequenz sollte daher so geregelt werden, daß das Stimulationsintervall bei jeder Belastung im Bereich des Plateauwertes $\text{APD}_{P_1}$ liegt.

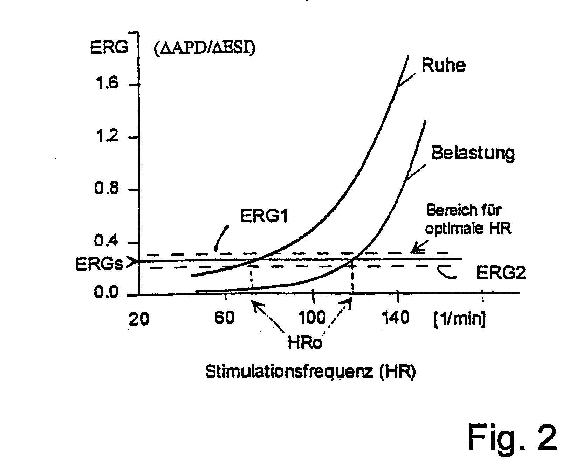

Um eine einfachere Größe für die Regelung verwenden zu können, wird vorteilhafterweise nicht direkt der Bereich um den Plateauwert selber gewählt, sondern der Gradient der Restitutionskurve. Der Gradient der Restitutionskurve im jeweiligen optimalen Arbeitspunkt, der durch die optimale Basiszykluslänge BCLo gegeben ist, ergibt sich dadurch, daß das extrasystolische. Intervall ESI prozentual ($\Delta$ESI/BCL) um einen definierten positiven +$\Delta$ESI und/oder negativen Wert - $\Delta$ESI verändert wird und die durch Pfeile 20 in FIG. 1 dargestellte, resultierende Veränderung der Aktionspotentialdauer +$\Delta$APD bzw. -$\Delta$APD gemessen wird. Wird dieser Gradient der elektrischen Restitution ERG = +$\Delta$APD/+$\Delta$ESI bzw. ERG = -$\Delta$APD/-$\Delta$ESI als Funktion der Stimulationsfrequenz HR für die Ruhe- und eine Belastungsphase aufgetragen, so ergibt sich der in FIG. 2 dargestellte Verlauf.

FIG. 2 zeigt, daß der exponentielle Anstieg des Gradienten der elektrischen Restitution ERG als Funktion einer steigenden Stimulationsfrequenz HR bei ansteigender Belastung nach rechts verschoben wird. Es ist zu erkennen, daß bei der jeweils optimalen Herzfrequenz die zugehörigen ERGo-Werte, die den Plateauwerten $\text{APD}_{P_1}$ in FIG. 1 entsprechen, etwa den gleichen Pegel haben, wobei jedoch die Werte auch unterschiedlich sein können. Diese Werte können bei einer Frequenzregelung als Sollwerte des Gradienten der elektrischen Restitution ERGs gewählt werden, wobei in FIG. 2 ein Bereich um den Sollwert ERGs herum als Bereich für eine optimale Stimulationsfrequenz HR angegeben ist, der durch die Schwellenwerte ERG1 und ERG2 begrenzt ist.

Es ist auch denkbar, daß der Gradient der elektrischen Restitution ERG aus der Differenz der positiven und negativen Änderung der Aktionspotentialdauer in Bezug auf die positive und negative Intervalländerung bestimmt wird, nämlich zu ERG = [(+$\Delta$APD)-(- $\Delta$APD)]/[(+$\Delta$ESI)-(-$\Delta$ESI)].

Anhand der FIGN. 1 und 2 läßt sich erkennen, daß die elektrische Restitutionsfunktion bzw. ihr Gradient ERG die Voraussetzung für die Regelung der Stimulationsfrequenz bietet, da einerseits der Gradient der elektrischen Restitution ERG bei Anstieg der Stimulationsfrequenz umgekehrt zum Anstieg der körperlichen Belastung reagiert und andererseits innerhalb eines physiologisch fest definierten Bereichs einen für jede Belastungssituation optimalen Wert ERGo aufweist. Aus der ERG-Charakteristik nach FIG. 2 ist zu erkennen, daß bei der Frequenzregelung eine zu hohe Stimulationsfrequenz (Overpacing) prinzipiell vermieden wird.

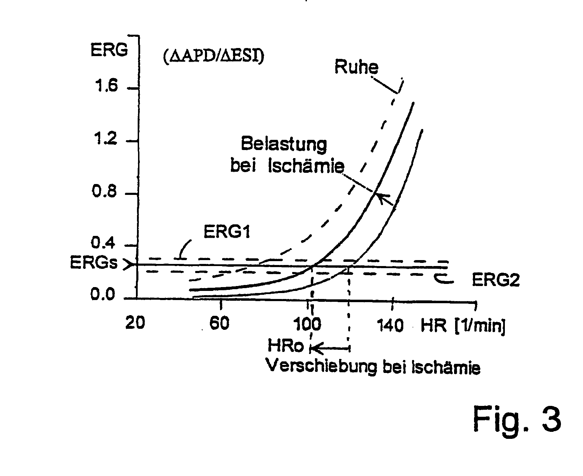

Es zeigt sich jedoch auch, daß eine bei Patienten mögliche akute Verschlechterung der myokardialen Leistung erkannt werden kann und bei der Frequenzanpassung berücksichtigt werden kann. In FIG. 3 ist der Gradient der elektrischen Restitution über die Stimulationsfrequenz für einen Fall dargestellt, bei dem eine Verschlechterung der myokardialen Leistung durch Ischämie auftritt. FIG. 3 zeigt, daß die Verlängerung des Stim-T-Intervalls beim Auftreten einer Ischämie im Belastungsfall die ERG-Kurve nach links verschiebt, d.h. der Gradient der elektrischen Restitution reagiert bei Abfall der myokardialen Leistungsfähigkeit wie bei einem Abfall der körperlichen Belastung. Als Folge davon wird die optimale Stimulationsfrequenz HRo reduziert und so die vorrangige Anforderung erfüllt, daß die ERG-abhängige Frequenzregelung bei einem sich krankhaft verschlechternden Herzmuskel ein Overpacing verhindert.

In einem anderen Ausführungsbeispiel kann auch anstelle des Gradienten die relative Änderung der elektrischen Restitution durch Bildung des Quotienten $\Delta$APD/$\Delta$ESI verwendet werden, wobei jeweils auch die Mittelwerte über mehrere Änderungszyklen bestimmt werden können.

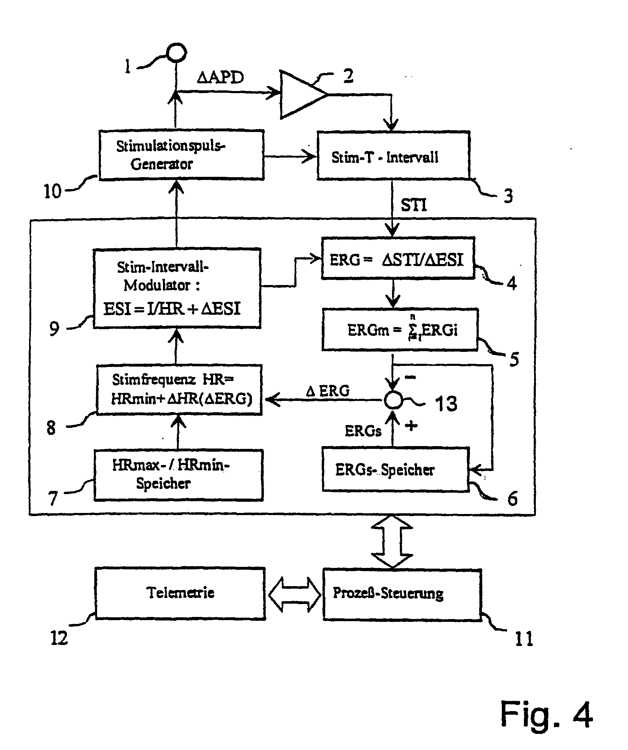

In FIG. 4 ist ein Ausführungsbeispiel eines Herzschrittmachers dargestellt, mit dem eine Frequenzregelung in Abhängigkeit von den Gradienten der elektrischen Restitutionsfunktion ERG verwendet wird.

Die für die ERG-abhängige Frequenz- bzw. Stimulationsintervallregelung benötigten Funktionsblöcke sind in dem umrandeten Feld dargestellt. Als andere Funktionsblöcke, die zur Standardausrüstung eines normalen QT-Schrittmachers gehören sind eine Stimulationselektrode 1 und ein die Stimulationselektrode 1 versorgender Stimulationspulsgenerator vorgesehen. Weiterhin ist ein EKG-Verstärker 2 einerseits mit der Stimulationselektrode 1 und andererseits mit einer Detektionsstufe zur Erfassung des Stim-T-Intervalls als Meßgröße verbunden. Außerdem enthält ein solches System einer über eine Telemetriestufe 12 programmierbaren Mikroprozessor mit einer Prozess-Steuerung 11.

Die Funktionsblöcke der Frequenzregelung sind ein HRmax/HRmin-Speicher 7 zur Speicherung der Grenzwerte der Stimulationsfrequenz, eine mit dem Speicher verbundene Regelstufe 8, der ein Regelwert $\Delta$ERG zugeführt wird, ein Stimulationsintervallmodulator 9 zum Festlegen und Modulieren des Stimulationsintervalls, der mit dem Stimulationspulsgenerator 10 verbunden ist. Weiterhin ist eine Rechnungsstufe 4 vorgesehen, die ein Signal von der Detektionsstufe 3 und vom Modulator 9 empfängt sowie eine Stufe 5 zur Bildung des Mittelwerts, ein Sollwertspeicher 6 und ein Soll-Ist-Wert-Vergleicher 13.

Die Funktionsweise des Herzschrittmachers ist wie folgt. Der Stimulationspulsgenerator 10 liefert ein Stimulationspuls an die Stimulationselektrode und oder EKG-Verstärker verstärkt das über die Stimulations-elektrode 1 abgeleitete intrakardiale EKG-Signal. Aus diesem verstärkten Signal analysiert die Detektionsstufe 3 die Intervalldauer STI zwischen Stimulationspuls und T-Welle, die dem QT-Intervall bzw. der Aktionspotentialdauer entspricht. In der Berechnungsstufe 4 wird der Gradient der-elektrischen Restitution ERG berechnet wobei jedoch auch andere oben erwähnte Größen verwendet werden können. Dazu wird zunächst, getriggert durch den Modulator 9, mit dem von der Detektionsstufe gelieferten Stim-T-Intervallwert die Änderung $\pm\Delta$STI berechnet, die durch die Änderung $\Delta$ESI des Stimulationsintervalls bewirkt wurde und anschließend wird der Quotient ERG = $\Delta$STI/$\Delta$ESI bestimmt. In der Mittelwertstufe 5 wird der Mittelwert ERGm der ERG-Werte über mehrere Änderungszyklen berechnet. Mit dem Pfeil vom Ausgang der Mittelwertstufe 5 zum Sollwertspeicher 6 wird angedeutet, daß der ERGm-Wert, der in körperlichem Ruhestand bei einer mittleren Stimulationsfrequenz von etwa 90/min gemessen wird, als Sollwert gespeichert wird.

In dem Soll-Istwert-Vergleich 13 wird die Differenz zwischen dem Mittelwert des Gradienten der elektrischen Restitution ERGm und dem Sollwert ERGs gebildet und als Differenzwert $\Delta$ERG an die Regelstufe 8 gegeben, wobei diese zur Einstellung der mittleren Stimulationsfrequenz $HR_0$ verwendet wird. Diese wird z.B. mit Hilfe folgender Funktionen berechnet:

$$HRo = HRmin + k*\Delta ERG$$

wobei HR so geregelt wird, daß HR < HRmax ist. Dabei sind HRmin und HRmax durch externe Programmierung vorgebbare und im Speicher 7 speicherbare Minimal- bzw. Maximalfrequenzen und k ein Proportionalitätsfaktor. HRmin wird üblicherweise durch die optimale mittlere Stimulationsfrequenz HRo-im Ruhezustand vorgegeben. Die so ermittelte Basisfrequenz HRo wird der Modulationsstufe 9 zugeführt, in der die Basiszykluslänge BCL = 1/HRo periodisch mit einer Intervalländerung $\pm\Delta$ESI moduliert wird und das resultierende Stimulationsintervall ESI = BCLo+ $\Delta$ESI gebildet wird. Im anschließenden Stimulationspulsgenerator 10 wird dann abhängig vom ESI-Wert der Stimulationspuls abgegeben. Die Regelung wird solange wiederholt bis der Wert $\Delta$ERG null ist.

In dem oben beschriebenen Beispiel wurde als Sollwert für den Gradienten der elektrischen Restitution ERGs der Pegel gewählt, der sich für die einzelnen Belastungskurven entsprechend FIG. 2 bei der optimalen Stimulationsfrequenz HRo ergibt, wobei Regelschwankungen zwischen den Werten ERG1 und ERG2 zugelassen sind. Der Sollwert ERGs kann aber auch automatisch an längerfristige Schwankungen des Restitutionsgradienten mit Hilfe eines zweiten, von der Modulation unabhängigen Meßparameters angepaßt werden, mit dem es möglich ist, den Ruhezustand des Patienten zu erkennen. In der Ruhephase wird dann automatisch die minimale Stimulationsrate HRmin eingestellt und der Sollwert ERGs an den in Ruhe gemessenen Restitutionsgradienten angepaßt. Auf diese Weise wird der Sollwert "nachkalibriert". Der von der Modulation unabhängige Meßparameter kann beispielsweise von einem mechanischen Bewegungssensor geliefert werden. Der Sollwert kann auch abhängig von der Frequenz eingestellt werden, beispielsweise kann er im Ruhezustand festgelegt werden und dann mit einer frequenzabhängigen Neigung versehen werden.

**Patentansprüche**

1. Herzschrittmacher mit einer Vorrichtung zum Erzeugen von mit einer mittleren Stimulationsintervalldauer aufeinander folgenden Stimulationspulsen, einer Vorrichtung zur Modulation einzelner Stimulationsintervalle (ESI) und einer Vorrichtung zur Erfassung eines Herzfunktionsparameters und zur Auswertung der durch die Modulation hervorgerufenen Änderungen des Herzfunktionsparameters, wobei abhängig von dem ausgewerteten Herzfunktionsparameter die Stimulationsintervalldauer geändert wird, und die Vorrichtung zur Modulation die Stimulationsintervalle (ESI) abwechseld so verkürzt (-$\Delta$ESI) und verlängert (+$\Delta$ESI), daß sich die jeweils eingestellte mittlere Stimulationsintervalldauer nicht ändert,
**dadurch gekennzeichnet,**
**daß** die Auswertevorrichtung die elektrische Restitution des Herzens bei dieser mittleren Stimulationsintervalldauer auf der Basis der Messung der Dauer des Aktionspotentials bestimmt, wobei die aufgrund der Modulation einzelner Stimulationsintervalle (ESI) auftretenden Änderungen einer Meßgröße der Dauer des Aktionspotentials gegenüber der bei der mittleren Stimulationsintervalldauer bestimmt werden und mit mindestens einem Sollwert (ERGs) verglichen werden und daß abhängig von dem Vergleich die mittlere Stimulationsintervalldauer geregelt wird.

2. Herzschrittmacher nach Anspruch 1, **dadurch gekennzeichnet, daß** die Vorrichtung zur Modulation einzelner Stimulationsintervalle (ESI) die abwechselnde Änderung im Abstand von mehreren Pulsen periodisch wiederholt durchführt.

3. Herzschrittmacher nach Anspruch 1, **dadurch gekennzeichnet, daß** die Vorrichtung zur Modulation einzelner Stimulationsintervalle (ESI) die abwechselnde Änderung (-$\Delta$ESI, +$\Delta$ESI) kontinuierlich durchführt.

4. Herzschrittmacher nach einem der Ansprüche 1 bis 3, **dadurch gekennzeichnet, daß** als Meßgröße zur Bestimmung der elektrische Restitution die Dauer des Aktionspotentials (ADP) des Herzmuskels oder das zeitliche Intervall zwischen Stimulationspuls und T-Welle im EKG (ST) oder QRS-Komplex und T-Welle im EKG (QT) verwendet wird.

5. Herzschrittmacher nach einem der Ansprüche 1 bis 4, **dadurch gekennzeichnet, daß** der Mittelwert der die elektrische Restitution bestimmenden Meßgröße (APDm, STm oder QTm) über mehrere Stimulationsintervalle errechnet wird.

6. Herzschrittmacher nach einem der Ansprüche 1 bis 5, **dadurch gekennzeichnet, daß** die von der jeweiligen Änderung des Stimulationsintervalls ($\Delta$ESI) abhängigen Änderungen der die elektrische Restitution bestimmenden Meßgröße gespeichert werden und daß über mehrere Änderungszyklen ihr Mittelwert ($\Delta$APDm bzw. $\Delta$STm oder $\Delta$QTm) bestimmt wird.

7. Herzschrittmacher nach einem der Ansprüche 1 bis 6, **dadurch gekennzeichnet, daß** zur Auswertung der Änderung der Meßgröße eine dimensionsfreie Größe der elektrischen Restitution genutzt wird.

8. Herzschrittmacher nach Anspruch 7, **dadurch gekennzeichnet, daß** als dimensionsfreie Größe der elektrischen Restitution der Gradient der elektrischen Restitution (ERG) durch Bildung des Quotienten $\Delta$APDM/$\Delta$ESI bzw. $\Delta$STm/$\Delta$ESI oder $\Delta$QTlm/$\Delta$ESI oder die relative Änderung der elektrischen Restitution durch Bildung des Quotienten $\Delta$APDm/APDm bzw. $\Delta$STm/STm oder $\Delta$QTm/QTm berechnet wird.

9. Herzschrittmacher nach Anspruch 1 bis 8, **dadurch gekennzeichnet, daß** der Sollwert (ERGs) durch den Wert des Gradienten bzw. der relativen Änderung der elektrischen Restitution im körperlichen Ruhezustand vorgegeben ist.

10. Herzschrittmacher nach Anspruch 5, **dadurch gekennzeichnet, daß** zur Anpassung des Sollwertes (ERGs) an individuelle Schwankungen der elektrischen Restitution die mittlere Stimulationsintervalldauer durch externe Programmierung im Ruhezustand des Patienten festgelegt wird und der in dieser Ruhephase gemessene Wert als absoluter Sollwert (ERGs) gespeichert wird.

11. Herzschrittmacher nach Anspruch 9, **dadurch gekennzeichnet, daß** zur Anpassung des Sollwertes (ERGs) an längerfristige Schwankungen der elektrischen Restitution mittels eines Sensors der Ruhezustand des Patienten erkannt wird und die mittlere Stimulationsintervalldauer eingestellt wird und der gespeicherte Sollwert (ERGs) durch den in der erfaßten Ruhephase gemessenen Wert ersetzt wird.

12. Herzschrittmacher nach einem der Ansprüche 1 bis 9, **dadurch gekennzeichnet, daß** zur Kompensation der frequenzabhängigen Änderungen bei der Detektion der T-Wellen der Sollwert (ERGs) abhängig von der Stimulationsintervalldauer geändert wird.

13. Herzschrittmacher nach einem der Ansprüche 1 bis 8, **dadurch gekennzeichnet, daß** die mittlere Stimulationsintervalldauer derart geregelt wird, daß sie ansteigt, wenn die Differenz zwischen dem Restitutionsgradienten und dem Sollwert einen negativen Schwellenwert unterschreitet und abfällt, wenn die Differenz einen positiven Schwellenwert überschreitet.

## Claims

1. A cardiac pacemaker having a device for generation of stimulation pulses succeeding one another at a mean stimulation interval duration, a device for modulation of single stimulation intervals (ESI), and a device for recording of a cardiac function parameter and for evaluating the changes in the cardiac function parameter that are brought about by the modulation, the stimulation interval duration being changed as a function of the evaluated cardiac function parameter, and the device for modulation alternately shortening (-$\Delta$ESI) and extending (+$\Delta$ESI) the simulation intervals (ESI) in such a way that the mean stimulation interval duration set up at the time does not change, **characterised in that** the evaluation device determines the electrical restitution of the heart for the said mean stimulation interval duration on the basis of measuring the duration of the action potential, the changes occurring, on account of the modulation of single stimulation intervals (ESI), in a measured variable of the duration of the action potential in relation to that for the mean stimulation interval period being determined and compared with at least one specified value (ERGs), and **in that** the mean stimulation interval duration is regulated as a function of the comparison.

2. A cardiac pacemaker according to Claim 1, **characterised in that** the device for modulation of single stimulation intervals (ESI) performs the alternating change periodically repeatedly at an interval of several pulses.

3. A cardiac pacemaker according to Claim 1, **characterised in that** the device for modulation of single stimulation intervals (ESI) performs the alternating change (-$\Delta$ESI, +$\Delta$ESI) continuously.

4. A cardiac pacemaker according to any one of Claims 1 to 3, **characterised in that** the duration of the action potential (ADP) of the myocardium or the interval in time between stimulation pulse and T wave in the ECG (ST) or QRS complex and T-wave in the ECG (QT) is used as measured variable for determination of electrical restitution.

5. A cardiac pacemaker according to any one of Claims 1 to 4, **characterised in that** the mean value of the measured variable (APDm, STm or QTm) determining electrical restitution is calculated over several stimulation intervals.

**6.** A cardiac pacemaker according to any one of Claims 1 to 5, **characterised in that** the changes in the measured variable determining electrical restitution, as a function of the respective change in the stimulation interval ($\Delta$ESI), are stored, and **in that** their mean value ($\Delta$APDm, $\Delta$STm or $\Delta$QTm) is determined over several change cycles.

**7.** A cardiac pacemaker according to any one of Claims 1 to 6, **characterised in that** a non-dimensional variable of electrical restitution is used to evaluate the change in the measured variable.

**8.** A cardiac pacemaker according to Claim 7, **characterised in that**, as non-dimensional variable of electrical restitution, the gradient of electrical restitution (ERG) is calculated by producing the quotient $\Delta$APDm/$\Delta$ESI, $\Delta$STm/$\Delta$ESI or $\Delta$QTIm/$\Delta$ESI or the relative change in electrical restitution is calculated by producing the quotient $\Delta$APDm/APDm, $\Delta$STm/STm or $\Delta$QTm/QTm.

**9.** A cardiac pacemaker according to Claims 1 to 8, **characterised in that** the specified value (ERGs) is given by the value of the gradient of or of the relative change in electrical restitution with the body at rest.

**10.** A cardiac pacemaker according to Claim 5, **characterised in that**, in order to adapt the specified value (ERGs) to individual fluctuations in electrical restitution, the mean simulation interval duration is determined by means of external programming when the patient is at rest, and the value measured in the said rest phase is stored as absolute specified value (ERGs).

**11.** A cardiac pacemaker according to Claim 9, **characterised in that**, in order to adapt the specified value (ERGs) to longer-term fluctuations in electrical restitution, the rest state of the patient is recognised by means of a sensor and the mean stimulation interval duration is set, and the stored specified value (ERGs) is replaced by the value measured in the recorded rest phase.

**12.** A cardiac pacemaker according to any one of Claims 1 to 9, **characterised in that**, in order to compensate for the frequency-dependent changes associated with the detection of T waves, the specified value (ERGs) is changed as a function of the stimulation interval duration.

**13.** A cardiac pacemaker according to any one of Claims 1 to 8, **characterised in that** the mean stimulation interval duration is regulated in such a way that it increases when the difference between the restitution gradient and the specified value goes below a negative threshold value and reduces when the difference exceeds a positive threshold value.

## Revendications

**1.** Stimulateur cardiaque comprenant un dispositif de production d'impulsions de stimulation qui se succèdent à une durée moyenne d'intervalle de stimulation, un dispositif de modulation des divers intervalles (ESI) de stimulation et un dispositif de détection d'un paramètre fonctionnel du coeur et d'évaluation des modifications, provoquées par la modulation, du paramètre fonctionnel du coeur, dans lequel, en fonction du paramètre fonctionnel du coeur qui est évalué, la durée de l'intervalle de stimulation est modifiée, et le dispositif de modulation écourte (-$\Delta$ESI) et prolonge (+$\Delta$ESI) , en alternance, les intervalles (ESI) de stimulation de façon à ce que la durée moyenne réglée de l'intervalle de stimulation ne soit pas modifiée,

**caractérisé en ce que** le dispositif d'évaluation détermine la restitution électrique du coeur pour cette durée moyenne d'intervalle de stimulation sur la base de la mesure de la durée du potentiel d'action, les modifications, se produisant sur la base de la modulation des divers intervalles (ESI) de stimulation, d'une grandeur de mesure de la durée du potentiel d'action par rapport à la durée moyenne de l'intervalle de stimulation, étant déterminées et étant comparées à au moins une valeur (ERGs) de consigne et la durée moyenne de l'intervalle de stimulation étant réglée en fonction de la comparaison.

**2.** Stimulateur cardiaque suivant la revendication 1, **caractérisé en ce que** le dispositif de modulation de divers intervalles (ESI) de stimulation effectue la variation alternée de manière périodiquement répétée à la distance de plusieurs impulsions.

**3.** Stimulateur cardiaque suivant la revendication 1, **caractérisé en ce que** le dispositif de modulation de divers intervalles (ESI) de stimulation effectue en continu la variation (-$\Delta$ESI, +$\Delta$ESI) alternée.

**4.** Stimulateur cardiaque suivant la revendication 1 à 3, **caractérisé en ce qu'**il est utilisé, comme grandeur de mesure pour la détermination de la restitution électrique, la durée du potentiel (ADP) d'action du muscle cardiaque, ou l'intervalle de temps entre une impulsion de stimulation et l'onde T dans l'ECG (ST) ou le complexe (QRS) et l'onde T dans l'ECG (QT).

**5.** Stimulateur cardiaque suivant l'une des revendications 1 à 4, **caractérisé en ce que** la valeur moyenne des grandeurs (APDm, STm ou QTm) de mesure déterminant la restitution électrique est calculée sur plusieurs intervalles de stimulation.

**6.** Stimulateur cardiaque suivant l'une des revendications 1 à 5, **caractérisé en ce que** les variations, en fonction des variations respectives de l'intervalle (ΔESI) de stimulation, des grandeurs de mesure déterminant la restitution électrique sont mémorisées et **en ce que** leur valeur (ΔAPDm, ou ΔSTm ou ΔQTm) moyennes sont déterminées sur plusieurs cycles de variation .

**7.** Stimulateur cardiaque suivant l'une des revendications 1 à 6, **caractérisé en ce que**, pour l'évaluation de la variation de grandeur de mesure, il est utilisé une grandeur sans dimension de la restitution électrique.

**8.** Stimulateur cardiaque suivant la revendication 7, **caractérisé en ce qu'**il est calculé comme grandeur sans dimension de la restitution électrique le gradient de la restitution (ERG) de la restitution électrique en formant le quotient ΔAPDM/ ΔESI ou ΔSTm/ ΔESI ou ΔQTIm/ ΔESI ou la variation relative de la restitution électrique en formant le quotient ΔAPDm/APDm ou ΔSTm/STm ou ΔQTm/QTm.

**9.** Stimulateur cardiaque suivant l'une des revendications 1 à 8, **caractérisé en ce que** la valeur (ERGs) de consigne est prescrite par la valeur du gradient ou de la variation relative de la restitution électrique à l'état de repos du corps.

**10.** Stimulateur cardiaque suivant la revendication 5, **caractérisé en ce que**, pour adapter la valeur (ERGs) de consigne aux fluctuations individuelles de restitution électrique, la durée moyenne de l'intervalle de stimulation est fixée par une programmation extérieure à l'état de repos du patient et la valeur mesurée dans cette phase de repos est mémorisée en tant que valeur ERGs absolue de consigne.

**11.** Stimulateur cardiaque suivant la revendication 9, **caractérisé en ce que**, pour adapter la valeur (ERGs) de consigne aux fluctuations de longue durée de la restitution électrique, on reconnaît au moyen d'un capteur l'état de repos du patient et on règle la durée moyenne de l'intervalle de stimulation et on remplace la valeur (ERGs) de consigne mémorisée par la valeur mesurée dans la phase de repos détectée.

**12.** Stimulateur cardiaque suivant l'une des revendications 1 à 9, **caractérisé en ce que**, pour compenser des variations en fonction de la fréquence lors de la détection des ondes T, la valeur (ERGs) de consigne est modifiée en fonction de la durée de l'intervalle de stimulation.

**13.** Stimulateur cardiaque suivant l'une des revendications 1 à 8, **caractérisé en ce que** la durée moyenne de l'intervalle de stimulation est réglée de façon à ce qu'elle augmente lorsque la différence entre le gradient de restitution et la valeur de consigne devient inférieure à une valeur de seuil négative et diminue lorsque la différence dépasse une valeur de seuil positive.

Fig. 1

Fig. 2

Fig. 3

**Fig. 4**